# FASCICULE DE BREVET EUROPEEN

(11) **EP 0 782 629 B1**
(45) Date de publication et mention de la délivrance du brevet: **02.12.1998**
(21) Numéro de dépôt: 95931259.6
(22) Date de dépôt: 19.09.1995
(51) Int. Cl.: C12P 11/00

(54) **HYDROLYSE ENZYMATIQUE DES 4-METHYLTHIOBUTYRONITRILES**
ENZYMATISCHE HYDROLYSE VON DEN 4-METHYLTHIOBUTYRONITRILEN
ENZYMATIC HYDROLYSIS OF 4-METHYLTHIOBUTYRONITRILES

(30) Priorité: 22.09.1994 FR 9411301; 07.03.1995 FR 9502615
(43) Date de publication de la demande: 09.07.1997
(73) Titulaire: RHONE-POULENC NUTRITION ANIMALE, F-92160 Antony (FR)
(72) Inventeur: FAVRE-BULLE, Olivier, F-69007 Lyon (FR); BONTOUX, Marie-Claude, F-38200 Luzinay (FR); LARGEAU, Denis, F-69390 Vourles (FR); ARIAGNO, André, F-69340 Francheville (FR)
(74) Mandataire: Savina, Jacques
(86) Numéro de dépôt international: FR9501196
(87) Numéro de publication internationale: WO9609403

(56) Documents cités:
- EP-A- 0 348 901
- EP-A- 0 444 640
- EP-A- 0 610 048
- EP-A- 0 610 049
- CHEMICAL ABSTRACTS, vol. 116, no. 25, 22 Juin 1992 Columbus, Ohio, US; abstract no. 254075, ENDO, RYUICHI ET AL 'Fermentative manufacture of.alpha.-hydroxy-4- methylthiobutyric acid' & JP,A,04 040 898 (NITTO CHEMICAL INDUSTRY CO., LTD., JAPAN)
- CHEMICAL ABSTRACTS, vol. 117, no. 5, 3 Août 1992 Columbus, Ohio, US; abstract no. 46744, ENDO, RYUICHI ET AL 'Fermentative manufacture of.alpha.-hydroxy-4-methylthiobutylamide' & JP,A,04 040 899 (NITTO CHEMICAL INDUSTRY CO., LTD., JAPAN)

## Description

La présente invention concerne l'utilisation d'une nitrilase comme catalyseur d'hydrolyse d'un groupe nitrile en groupe carboxylique. Elle concerne plus précisement l'utilisation de la nitrilase choisie parmi celles *d'Alcaligenes faecalis* déposée sous le numéro ATCC 8750, de *Rhodococcus sp.* HT 29-7 déposée sous le numéro FERM BP-3857 ou de *Gordona terrae* MA-1 déposée sous le numéro FERM BP-4535.

La nitrilase *d'Alcaligenes faecalis* est décrite par exemple dans les brevets européens et japonais publiés sous les numéros respectifs EP 348 901 et JP 03 224 496, tous deux appartenant à la société ASAHI. Dans ces brevets, et particulièrement dans le brevet européen, la nitrilase est utilisée pour produire à partir de nitriles racémiques des acides optiquement actifs. Les nitriles préférés de départ sont a substitués et comportent une chaîne alkyle contenant de préférence 1 à 3 atomes de carbone ou un radical aromatique. L'exemple 11 du brevet européen sus mentionné décrit l'hydrolyse du mandélonitrile racémique par *Alcaligenes faecalis,* l'acide mandélique R-(-) est obtenu avec un excès énantiomérique de 91 %. Le brevet européen 486 289 et son équivalent américain le brevet US 5 326 702 de la société NITTO CHEMICAL décrivent l'utilisation *d'Alcaligenes sp* BC35-2 (FERM No 11265 ou FERM-BP-3318) pour hydrolyser le mandélonitrile en présence d'un sulfite, l'acide mandélique est obtenu dans ce cas avec un excès énantiomérique d'environ 98%.

Le brevet JP 04 341 185 de la société ASAHI décrit encore la préparation et l'utilisation de la nitrilase *d'Alcaligenes faecalis* ATCC 8750 pour résoudre les problèmes de préparation de composés optiquement purs à partir de leur nitrile racémique. La nitrilase énantiosélective décrite dans le brevet japonais ci-dessus référencé est capable plus préférentiellement d'hydrolyser les 2-hydroxynitrile de formule générale (I) suivante en acide 2-hydroxycarboxylique de formule (Il) suivante : 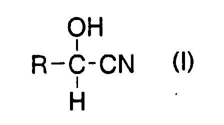 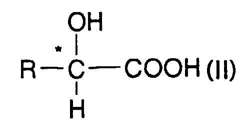 dans lesquelles R représente un groupe aryle éventuellement substitué ou un groupe hétérocyclique éventuellement substitué. Il est indiqué dans ce brevet que l'hydrolyse du nitrile de l'acide mandélique par ladite nitrilase permet d'obtenir 100 % d'excès énantiomérique en acide R-(-) mandélique. Il est indiqué dans l'exemple 5 que ladite nitrilase peut être utilisée pour hydrolyser des nitriles aliphatiques comme le valéronitrile, l'acrylonitrile, les 2-halogénopropionitriles, le chloroacétonitrile. Rien n'est précisé en ce qui concerne l'énantiosélectivité de cette hydrolyse sur les nitriles aliphatiques ayant un centre d'asymétrie.

Ce texte précise aussi que la nitrilase *d'Alcaligenes faecalis* présente un optimum d'activité à un pH compris entre 6.5 et 8.0, la température de mise en oeuvre de la dite enzyme doit toujours, selon cette référence, être située en-dessous de 45°C.

Il est apparu de façon tout à fait étonnante que ladite enzyme *d'Alcaligenes faecalis* ATCC 8750, lorsqu'elle était utilisée pour hydrolyser le 4-méthylthio 2-hydroxy butyronitrile, réalisait cette hydrolyse sans aucune sélectivité du point de vue de la pureté optique. Cette enzyme hydrolyse ledit nitrile en produisant un mélange racémique des deux acides sans aucune prépondérance de l'un ou de l'autre des isomères.

La nitrilase de *Rhodococcus sp.* HT 29-7 déposée sous le numéro FERM BP-3857 est décrite par exemple dans les brevets européen et américain publiés sous les numéro respectifs EP 610 049 et US 5 296 373, tous deux appartenant à la société NITTO.

Dans ces brevets, et particulièrement dans le brevet américain, la nitrilase est utilisée pour produire à partir de nitriles racémiques porteur d'un groupe phényl des acides optiquement actifs. Les nitriles de départ comportent tous un radical aromatique, il s'agit essentiellement de l'hydrolyse du mandélonitrile ou de ses dérivés substitués sur le noyau aromatique. L'exemple 1 du brevet américain sus mentionné décrit l'hydrolyse du mandélonitrile racémique par *Rhodococcus sp.* HT 29-7, l'acide mandélique R-(-) est obtenu avec un excès énantiomérique de 100 %. L'exemple 2 décrit l'hydrolyse des dérivés substitués du mandélonitrile sur le noyau, l'excès énantiomérique en dérivé de l'acide mandélique est aussi de 100 %, il en est de même lorsque l'hydrolyse est réalisée sur les nitriles du benzaldéhyde.

Le brevet européen 610 049 de la société NITTO CHEMICAL décrit l'utilisation de *Rhodococcus sp.* HT 29-7 (FERM BP-3857), *d'Alcaligenes sp*. BC35-2 (FERM BP-3318) ou de *Brevibacterium acetylicum* IAM 1790 pour hydrolyser des α hydroxy nitriles substitués en position γ par un noyau aromatique en acide a hydroxycarboxylique substitué par un groupe phényle optiquement actif. Les acides obtenus avec *Rhodococcus sp.* HT 29-7 présentent toujours un excès énantiomériques variable selon le nitrile de départ et selon la présence ou l'absence d'acide phosphorique (pH fixé aux environs de 8,2).

Il est apparu de façon tout à fait étonnante que ladite enzyme de *Rhodococcus sp.* HT 29-7 (FERM BP-3318), lorsqu'elle était utilisée pour hydrolyser le 4-méthylthio 2-hydroxy butyronitrile, réalisait cette hydrolyse sans aucune sélectivité du point de vue énantiomérique. Cette enzyme hydrolyse ledit nitrile en produisant un mélange racémique des deux acides sans aucune prépondérance de l'un ou de l'autre des isomères.

La nitrilase de *Gordona terrae* MA-1 déposée sous le numéro FERM BP-4535 est décrite dans le brevet européen 0 610 048 pour hydrolyser des nitriles porteurs en γ d'un groupe phényl et en α d'un groupe hydroxy en acide correspondant optiquement actif. L'excès énantiomérique varie dans tous les exemples entre 92 et 100%.

Il est apparu de façon tout à fait étonnante que ladite enzyme de *Gordona terrae* MA-1 déposée sous le numéro FERM BP-4535, lorsqu'elle était utilisée pour hydrolyser le 4-méthylthio 2-hydroxy butyronitrile, réalisait cette hydrolyse sans aucune sélectivité du point de vue énantiomérique. Cette enzyme hydrolyse ledit nitrile en produisant un mélange racémique des deux acides sans aucune prépondérance de l'un ou de l'autre des isomères.

La présente invention concerne donc un procédé de préparation d'acides 4-méthylthio butyrique α substitués racémiques par hydrolyse des 4-méthylthio butyronitrile α substitués racémiques par la nitrilase d'un des microorganismes suivants: *Alcaligenes faecalis* déposé sous le numéro ATCC 8750, *Rhodococcus sp.* HT 29-7 déposée sous le numéro FERM BP-3857 ou de *Gordona terrae* MA-1 déposée sous le numéro FERM BP-4535.

Selon le procédé de l'invention, le micro-organisme peut être utilisé tel quel ou immobilisé sur des supports bien connus de l'homme de l'art.

Par ailleurs l'information génétique qui code pour l'enzyme peut être transférée du micro-organisme parental (tel que *A. faecalis* ATCC8750...) vers un micro-organisme tel que *Bacillus subtilis.*

Une variante du procédé de l'invention consiste à mettre en oeuvre en quantité correspondante, à la place d'un micro-organisme, son enzyme libre ou immobilisée, celle-ci étant totalement ou partiellement purifiée.

On préfère parmi les 4-méthylthio butyronitriles a substitués utiliser le 4-méthylthio 2-hydroxy butyronitrile qui permet de préparer l'hydroxyanalogue de la méthionine racémique. Cette technologie de préparation des dérivés de la méthionine permet d'éviter la formation de produits secondaires minéraux en quantités importantes dont les rejets posent de plus en plus de problèmes à cause des contraintes en ce qui concerne la protection de l'environnement.

Ces enzymes présentent dans le cadre de la présente invention une activité nitrilase dans une zone de pH comprise entre 4 et 11 et un optimum d'activité à un pH compris entre 5 et 9. Leur température optimale de mise en oeuvre est comprise entre 30 et 60°C, on préfère néanmoins les utiliser entre 30 et 50°C.

On préfère travailler avec une concentration en nitrile dans la solution de départ comprise entre 10 mmoles et 400 mmoles par litre et de préférence entre 50 et 200 mmoles par litre. La concentration en sel d'ammonium de l'acide obtenu a peu d'influence sur l'activité de l'enzyme lorsqu'elle est inférieure à 2 moles par litre et elle est de préférence comprise entre 0,1 et 1,5 mole par litre.

L'invention sera plus complètement décrite à l'aide des exemples suivants qui ne doivent pas être considérés comme limitatifs de l'invention.

### EXEMPLE 1

Le micro-organisme *Alcaligenes faecalis* ATCC 8750 est cultivé dans le milieu suivant:

| | |
|---|---|
| Acétate d'ammonium | 10 g/l |
| Extrait de levure | 5 g/l |
| Peptone | 5 g/l |
| K₂HPO₄ | 5 g/l |
| MgSO₄, 7H₂O | 0,2 g/l |
| FeSO₄, 7H₂O | 30 mg/l |
| NaCI | 1 g/l |
| Benzonitrile | 0,5 g/l |
| pH 7,2 | |

La culture est réalisée à 30°C dans des fioles d'Erlenmeyer de 2 litres contenant 600 ml de milieu. Le tout est agité à 150 tours par minute durant 30 heures. Le culot cellulaire est récupéré, lavé avec une solution de NaCI à 9g/l puis congelé.

Conditions opératoires pour l'hydrolyse du butyronitrile :

| | |
|---|---|
| 4-méthylthio 2-hydroxy butyronitrile | 50 mM |
| Cellules | 5 mg/ml |
| Tampon phosphate | 200 mM |
| Température | 30°C |
| Durée | 4 heures |

L'étude cinétique d'hydrolyse du 4-méthylthio 2-hydroxy butyronitrile montre une production linéaire de 2,8 µmoles d'hydroxy analogue de la méthionine par heure et par mg de cellules sèches. L'excès énantiomérique de l'acide formé a été mesuré par chromatographie liquide, il est égal à 0 % pour un rendement en acide de 80 %.

### EXEMPLE 2

On a évalué la stabilité de l'enzyme en suivant la cinétique de production de l'hydroxy analogue de la méthionine pendant 180 heures avec des cellules libres.

Les conditions opératoires sont les suivantes :

| | |
|---|---|
| 4-méthylthio 2-hydroxy butyronitrile | 100 mM |
| Cellules | 10 mg/ml |
| Tampon phosphate 300 mM | 5 ml |
| Température | 25°C |

Le nitrile est ajouté séquentiellement par ajout de 100mmoles de nitrile après épuisement. La formation de l'acide correspondant est indiqué dans le tableau 1:

| TEMPS | Acide Formé (mmoles/litre) | Activité (mmoles/h. mg de cell. s. |
|---|---|---|
| O | 0 | 0 |
| 2 h | 39 | 1,7 |
| 17 h | 230 | 1,3 |
| 24 h | 312 | 1,7 |
| 47 h | 466 | 1,2 |
| 70 h | 556 | 0,4 |
| 172 h | 860 | 0,6 |
| 180 h | 940 | 0,6 |

L'activité initiale est de 2 µmoles/h.mg de cellules sèches. Cet exemple prouve la stabilité de l'enzyme malgré la présence de fortes concentrations en acide (0,9 mole d'hydroxy analogue de la méthionine)

### EXEMPLE 3

La stabilité de l'enzyme en fonction de la quantité de substrat et de la quantité d'acide formé a aussi été mesurée dans les conditions suivantes :

### Stabilité de l'enzyme en fonction de la quantité de substrat

| | |
|---|---|
| Nitrile | cf Tableau résultats |
| Cellules | 10 mg/ ml |
| Tampon phosphate 300 mM pH 7,0 | 5 ml |
| Température | 25°C |
| Durée | 1 heure |

| Concentration en nitrile (mM) | Activité (µmoles/ h. mg de cell. s.) |
|---|---|
| 50 | 2 |
| 100 | 2 |
| 150 | 2 |
| 200 | 2 |
| 500 | 0 |

### Stabilité de l'enzyme en fonction de la quantité d'acide formé

Conditions opératoires:

| | |
|---|---|
| Nitrile | 50 mM |
| Cellules | 2,5 mg/ ml |
| Tampon phosphate 100 mM pH 7,0 | 1 ml |
| Température | 30°C |
| Durée | 2 heures |

| Concentration en acide (mM) | Activité (µmoles/ h. mg de cell. s.) |
|---|---|
| 0 | 3,2 |
| 100 | 4,5 |
| 200 | 3,8 |
| 300 | 4,1 |
| 400 | 4 |
| 500 | 4 |
| 600 | 3,4 |
| 700 | 2,8 |

### EXEMPLE 4

### Purification de la nitrilase

Les cellules *d'Alcaligenes faecalis* sont cultivées 24 heures à 30°C dans le milieu décrit à l'exemple 1.

Après centrifugation de la culture, le culot est repris dans le tampon pH 7,5 (25mM trisHCl, 10 % (p/v) glycérol). La suspension cellulaire est traitée aux ultrasons puis centrifugée afin d'obtenir l'extrait brut. L'extrait brut est ensuite traité avec du sulfate d'ammonium jusqu'à 30 % de la saturation. Le précipité obtenu est resuspendu dans le tampon pH 7,5 puis dialysé contre 2 litres du même tampon durant une nuit.

La solution obtenue est ensuite déposée sur une colonne échangeuse d'anions Q Sepharose Fast Flow HR 26/10® préalablement équilibrée avec le tampon pH 7,5. L'activité est ensuite éluée avec un gradient de 0 à 1 M de NaCI.

Les fractions actives sont ensuite déposées sur une colonne échangeuse d'anions Mono Q HR 5/5® préalablement équilibrée avec du tampon pH 7,5. La nitrilase est éluée à l'aide d'un gradient de 0 à 1 M de NaCI.

Pour finir, les fractions contenant l'activité sont réunies, puis est ajouté 1 M de sulfate d'ammonium. Cette solution est déposée sur une colonne d'intéractions hydrophobes Phényl sepharose HR 5/5® préalablement équilibrée avec le tampon pH 7,5 additioné de 1 M de (NH₄)₂SO₄. L'activité est ensuite éluée avec un gradient de 1 à 0 M en sulfate d'ammonium.

Le poids moléculaire de la protéine est déteminé par gel filtration. Il est d'environ 260 kDa. Sur gel de SDS-PAGE, on observe une unique bande de 43 kDa (pureté de 95 %).

La cinétique d'hydrolyse du 4-méthylthio 2-hydroxy butyronitrile en hydroxy analogue de la méthionine par la nitrilase d'*A. faecalis* est linéaire

| Temps (Heure) | Activité (µmoles/ h. mg de prot.) | RR (%) |
|---|---|---|
| 0 | 0 | 1 |
| 0,5 | 150 | 3 |
| 1 | 171 | 5 |
| 21,5 | 150 | 68 |
| 24,8 | 150 | 77 |
| 29 | 150 | 87 |

### EXEMPLE 5 Influence du pH

Conditions opératoires :

| | |
|---|---|
| nitrile | 50 mM |
| protéine | 50 µg/ml |
| tampon acétate 100 mM de pH 4 à 5 | |
| tampon phosphate 100 mM de pH 6 à 7 | 1 ml |
| tampon TRIS-HCI 100 mM de pH 8 à 9 | |
| tampon borate 100 mM de pH 10 à 11 | |
| température | 30°C |
| Durée | 1 à 2 heures |

| pH | Activité (µmoles/ h. mg de prot.) |
|---|---|
| 4 | 0 |
| 5 | 42 |
| 6 | 232 |
| 7 | 272 |
| 8 | 405 |
| 9 | 412 |
| 10 | 158 |
| 11 | 0 |

### EXEMPLE 6 Influence de la température

Conditions opératoires

| | |
|---|---|
| nitrile | 50 mM |
| protéine | 50 µg/ml |
| tampon phosphate100 mM de pH 7,0 | |
| températures variables de 4°C à 60°C | |
| durée | 1 heure |

La température optimale de fonctionnement de l'enzyme est de 50°C.

| Température en °C | Activité (µmoles/ h. mg de prot.) |
|---|---|
| 4 | 45 |
| 10 | 67 |
| 20 | 140 |
| 30 | 272 |
| 40 | 419 |
| 50 | 570 |
| 60 | 333 |

### EXEMPLE COMPARATIF AVEC LE MANDELONITRILE

Conditions opératoires:

| | |
|---|---|
| Mandélonitrile | 7 mM |
| Protéine | 5 µg/ ml |
| Tampon phosphate 100 mM pH 7,0 | 1 ml |
| Température | 30°C |

| Temps (minute) | Activité (µmoles/ h. mg de prot.) | ee |
|---|---|---|
| 15 | 1000 | 1 |
| 60 | 1030 | 1 |

La nitrilase purifiée est donc énantiosélective sur le mandélonitrile mais pas sur le 4-méthylthio 2-hydroxybutyronitrile.

### EXEMPLE 7

### Hydrolyse du 4-méthyl thio 2-hydroxy butyronitrile par Rhodococcus HT 29-7 FERM BP-3857

Le micro-organisme *Rhodococcus* sp. HT 29-7 FERM BP-3857 est cultivé dans le milieu suivant:

| | |
|---|---|
| Glycérol | 20 g/l |
| Extrait de levure (DIFCO) | 3 g/l |
| KH₂PO₄ | 1 g/l |
| Na₂HPO₄.12H₂O | 4,4 g/l |
| Na₂SO₄ | 2,8 g/l |
| MgCl₂.6H₂O | 0,85 g/l |
| CaCl₂.2H₂O | 0,05 g/l |
| MnSO₄.H₂O | 0,033 g/l |
| FeSO₄.7H₂O | 0,013 g/l |
| ZnSO₄.7H₂O | 0,005 g/l |
| Benzonitrile | 0,5 g/l |
| pH 7,5 | |

La culture est réalisée à 30°C dans des fioles d'Erlenmeyer de 2 litres contenant 600 ml de milieu. Le tout est agité à 150 tours par minute durant 140 heures. Le culot cellulaire est récupéré, lavé avec une solution NaCI à 9 g/l puis congelé.

### Influence du pH pour l'hydrolyse :

Conditions opératoires:

| | |
|---|---|
| 4-méthyl thio 2-hydroxy butyronitrile | 100 mM |
| Densité Optique à 660nm | 15 |
| Tampon acétate 100 mM de pH 4 à 5 | |
| Tampon phosphate 100 mM de pH 6,0 à 7,0 | 1 ml |
| Tampon TRIS-HCI 100 mM de pH 8,0 à 9,0 | |
| Tampon Borate 100 mM de pH 10,0 à 11,0 | |
| Température | 30°C |
| Durée | 10 heures |

| pH | Rendement en acide |
|---|---|
| 4 | 0% |
| 5 | 100% |
| 6 | 100% |
| 7 | 100% |
| 8 | 100% |
| 9 | 100% |
| 10 | 20% |
| 11 | 0% |

### EXEMPLE 8: Influence de la concentration en substrat.

La stabilité de l'enzyme en fonction de la quantité de substrat a aussi été mesurée dans les conditions suivantes:

| | |
|---|---|
| 4-méthyl thio 2-hydroxy butyronitrile | cf Tableau résultats |
| Densité Optique à 660nm | 20 |
| Tampon phosphate 300 mM pH 7,0 | 5 ml |
| Température | 30°C |
| Durée | 2 heures |

| Concentration en nitrile (mM) | Rendement en acide |
|---|---|
| 50 | 90% |
| 100 | 44% |
| 200 | 22% |
| 300 | 10% |
| 400 | 0% |
| 500 | 0% |

### EXEMPLE 9 Stabilité de l'enzyme en fonction de la quantité d'acide formé

Conditions opératoires:

| | |
|---|---|
| 4-méthyl thio 2-hydroxy butyronitrile | 100 mM |
| Densité Optique à 660nm | 15 |
| Tampon phosphate 100 mM pH 7,0 | 1 ml |
| Température | 30°C |
| Durée | 6 heures |

| Concentration en acide (mM) | rendement en acide |
|---|---|
| 0 | 100% |
| 200 | 100% |
| 400 | 100% |
| 600 | 100% |
| 800 | 100% |
| 1000 | 100% |

### EXEMPLE 10

Excès énantiomérique.

| | |
|---|---|
| 4-méthylthio 2-hydroxy butyronitrile | 140 mM |
| Densité Optique à 660nm | 14 |
| Tampon phosphate 100 mM pH 7,0 | 1 ml |
| Température | 20°C |

| Temps d'incubation | Rendement en acide | Pureté optique |
|---|---|---|
| 2h | 15% | 0 |
| 6 h | 50% | 0 |
| 24 h | 100% | 0 |

L'étude cinétique d'hydrolyse du 4-méthylthio 2-hydroxy butyronitrile montre une production linéaire d'hydroxy analogue de la méthionine par heure par mg de cellules sèches. L'excès énantiomérique de l'acide formé a été mesuré par chromatographie liquide, il est égal à 0% pour un rendement en acide variant de 15% à 100%.

### EXEMPLE 11 Influence de la température.

Conditions opératoires:

| | |
|---|---|
| 4-méthyl thio 2-hydroxy butyronitrile | 100 mM |
| Densité Optique à 660nm | 15 |
| Tampon phosphate 100 mM pH 7,0 | 1 ml |
| Température | voir tableau |
| Durée | 6 heures |

| T (°C) | rendement en acide |
|---|---|
| 10 | 70% |
| 20 | 100% |
| 30 | 100% |
| 40 | 100% |
| 50 | 31% |
| 60 | 15% |

### EXEMPLE 12 Hydrolyse du 4 méthylthio 2-aminobutyronitrile.

Conditions opératoires:

| | |
|---|---|
| 4-méthyl thio 2-hydroxy butyronitrile | 23 mM |
| Densité Optique à 660nm | 15 |
| Tampon phosphate 100 mM pH 7,0 | 1 ml |
| Température | 30°C |

| Temps (heure) | rendement en acide | excès énantiomérique de l'acide |
|---|---|---|
| 0,5 | 40% | n.d. |
| 1 | 51% | 0,12 |
| 2 | 62% | n. d. |
| 5 | 78% | 0,15 |

### EXEMPLE 13

Les conditions de cultures utilisées pour *Gordona terrae* MA-1 (FERM BP-4535) sont les suivantes :

| | |
|---|---|
| Glycérol | 10 g/l |
| Extrait de levure | 0,4 g/l |
| K₂HPO₄ | 6,8 g/l |
| Na₂HPO₄.12H₂O | 7,1 g/l |
| Na₂SO₄ | 2,8 g/l |
| MgCl₂.6H₂O | 0,4 g/l |
| CaCl₂.2H₂O | 40 mg/l |
| MnSO₄.H₂O | 4 mg/ ml |
| FeCl₃ | 0,6 mg/l |
| ZnSO₄ | 0,3 mg/l |
| Benzonitrile | 0,5 g/l |
| pH 7,2 | |

### Activité de l'enzyme

Les cellules cultivées sont ensuite lavées puis mises en présence de l'hydroxy méthylthio butyronitrile pour le dosage de l'activité

Conditions opératoires :
[nitrile] = 23 mM;
[cellules] = 6,8 g/l ;
tampon phosphate 100 mM
pH 7,0; 35°C

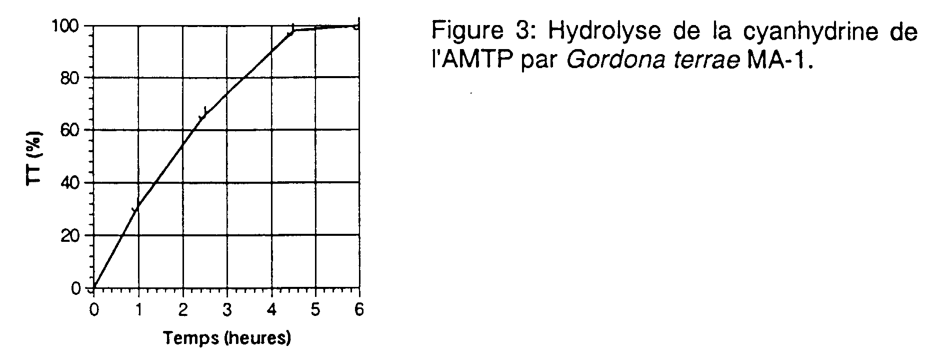

La cinétique d'hydrolyse de l'hydroxy méthylthio butyro nitrile est linéaire. La vitesse initiale est estimée à 13 mmol/ h. g CS .

### EXEMPLE 14 Influence de la concentration en cyanhydrine de l'AMTP sur l'activité nitrilase.

L'influence de la concentration initiale en hydroxy méthylthio butyronitrile sur l'activité nitrilase a été déterminée, les résultats sont indiqués dans le tableau suivant.

Conditions opératoires:
[cellules]=5,1 g/l ;
Tampon phosphate 100 mM
pH 7,0; 35°C.

La cinétique est réalisée sur 0,5, 1, 2 et 3 heures.

| Concentration en nitrile (mM) | Activité (mmol/ h. g CS) |
|---|---|
| 50 | 14 |
| 100 | 13 |
| 200 | 15 |
| 300 | 0 |
| 400 | 0 |

Jusqu'à 200 mM, l'activité varie peu avec la concentration en substrat.

### EXEMPLE 15 Influence de la concentration en hydroxy analogue de la méthionine sur l'activité nitrilase.

Dans cet essai, nous avons étudié l'influence de la concentration en hydroxy-4 méthylthio-2 butanoate d'ammonium sur l'activité nitrilase.

Condition opératoire:
[cellules] = 5 g/l;
[nitrile] = 100 mM;
tampon phosphate 100 mM pH 7,0;
35°C.

La concentration en carboxylate d'ammonium varie entre 0 et 1,5 M.

| Concentration en acide (mole/l) | Activité (µmol/ h. mg de CS) |
|---|---|
| 0 | 9,4 |
| 0,5 | 14 |
| 1 | 19 |
| 1,5 | 15 |

La concentration en hydroxy-4 méthylthio-2 butanoate d'ammonium ne modifie en rien l'activité initiale de la souche *Gordona terrae* HT29-7.

### EXEMPLE 16 Influence du pH et de la température.

Conditions opératoires:
[nitrile]=100 mM;
[cellules]=7,5 g/l;
Tampon acétate 100 mM pH 4 à 5;
tampon phosphate 100 mM pH 6 à 7;
tampon Tris-HCI 100 mM pH 8 à 9
tampon Borate 100 mM pH 10 à 11; 3
30°C; cinétique sur 1, 2, 4 et 6 heures.

| pH | Activité (mmol/ h. g CS) |
|---|---|
| 4 | 0,6 |
| 5 | 2,5 |
| 6 | 7,8 |
| 7 | 9,6 |
| 8 | 15,3 |
| 9 | 18 |
| 10 | 5,7 |
| 11 | 11 |

Le pH optimum se situe aux alentours de 8-9 dans nos conditions opératoires.

### Influence de la température sur l'activité nitrilase de Gordona terrae MA-1.

Conditions opératoires:
[nitrile]=100 mM;
[cellules]=7,5 g/l;
Tampon phosphate 100 mM pH 7,0;
cinétique sur 1 heure.

| T (°C) | Activité (mmol/ h. g CS) |
|---|---|
| 10 | 0,6 |
| 20 | 2,3 |
| 30 | 3,2 |
| 40 | 3,4 |
| 50 | 3,5 |
| 60 | 1,9 |

La température optimale se situe vers 40-50°C.

## Revendications

1. Procédé de préparation d'acides 4-méthylthio butyrique α substitués racémiques caractérisé en ce qu'on hydrolyse les 4-méthylthio butyronitrile α substitués racémiques par une nitrilase choisie parmi celles *d'Alcaligenes faecalis* ATCC-8750, *Rhodococcus sp.* HT 29-7 FERM BP-3857 ou de *Gordona terrae* FERM BP-4535.

2. Procédé selon la revendication 1 caractérisé en ce que le 4-méthylthio butyronitrile α substitués racémiques est le 4-méthylthio 2-hydroxy butyronitrile

3. Procédé selon la revendication 1 caractérisé en ce que le pH du milieu est compris entre 4 et 11 et de préférence entre 5 et 9.

4. Procédé selon la revendication 1 caractérisé en ce que la température d'hydrolyse est comprise entre 30 et 60°C et de préférence 30 et 50°C.

5. Procédé selon la revendication 1 caractérisé en ce que la concentration en 4-méthylthio butyronitrile α substitué dans la solution de départ est comprise entre 10 et 400 mmoles et de préférence entre 50 et 200 mmoles par litre.

6. Procédé selon la revendication 1 caractérisé en ce que la concentration en acide 4 méthylthio butyrique α substitué dans la solution est comprise entre 10 et 2000 mmoles par litre et de préférence entre 100 et 1500 mmoles par litre.

## Claims

1. Process for the preparation of racemic α-substituted 4-methylthiobutyric acids, characterized in that the racemic α-substituted 4-methylthiobutyronitriles are hydrolysed by a nitrilase chosen from those of *Alcaligenes faecalis* ATCC-8750, *Rhodococcus sp.* HT 29-7 FERM BP-3857 or *Gordona terrae* FERM BP-4535.

2. Process according to claim 1, characterized in that the racemic α-substituted 4-methylthiobutyronitrile is 4-methylthio-2-hydroxybutyronitrile.

3. Process according to claim 1, characterized in that the pH of the medium is between 4 and 11 and preferably between 5 and 9.

4. Process according to claim 1, characterized in that the hydrolysis temperature is between 30 and 60°C and preferably 30 and 50°C.

5. Process according to claim 1, characterized in that the concentration of α-substituted 4-methylthiobutyronitrile in the starting solution is between 10 and 400 mmol and preferably between 50 and 200 mmol per litre.

6. Process according to claim 1, characterized in that the concentration of α-substituted 4-methylthiobutyric acid in the solution is between 10 and 2000 mmol per litre and preferably between 100 and 1500 mmol per litre.

## Patentansprüche

1. Verfahren zur Herstellung von racemischen α-substituierten 4-Methylthiobuttersäuren, dadurch gekennzeichnet, daß man die racemischen α-substituierten 4-Methylthiobutyronitrile durch eine Nitrilase hydrolysiert, die unter denen von *Alcalingenes faecalis* ATCC8750, *Rhodococcus sp*. HT 29-7 FERM BP 3857 oder von *Gordona terrae* FERM BP4535 ausgewählt ist.

2. Verfahren gemäß Anspruch 1, dadurch gekennzeichnet, daß das racemische α-substituierte 4-Methylthiobutyronitril das 4-Methylthio-2-hydroxybutyronitril ist.

3. Verfahren gemäß Anspruch 1, dadurch gekennzeichnet, daß der pH-Wert des Mediums zwischen 4 und 11 und vorzugsweise zwischen 5 und 9 liegt.

4. Verfahren gemäß Anspruch 1, dadurch gekennzeichnet, daß die Hydrolysetemperatur zwischen 30 und 60 °C und vorzugsweise 30 und 50 °C liegt.

5. Verfahren gemäß Anspruch 1, dadurch gekennzeichnet, daß die Konzentration an α-substituiertem 4-Methylthiobutyronitril in der Ausgangslösung zwischen 10 und 400 mmol und vorzugsweise zwischen 50 und 200 mmol pro Liter liegt.

6. Verfahren gemäß Anspruch 1, dadurch gekennzeichnet, daß die Konzentration an α-substituierter 4-Methylthiobuttersäure in der Lösung zwischen 10 und 2000 mmol pro Liter und vorzugsweise zwischen 100 und 1500 mmol pro Liter liegt.
